**Europäisches Patentamt**

**European Patent Office**

**Office européen des brevets**

(19)

(11) Veröffentlichungsnummer: **0 086 890**
**B1**

(12) # EUROPÄISCHE PATENTSCHRIFT

(45) Veröffentlichungstag der Patentschrift:
**10.07.85**

(21) Anmeldenummer: **82111657.1**

(22) Anmeldetag: **25.01.83**

(51) Int. Cl.⁴: **C 07 C 7/06,** C 07 C 9/14,
C 07 C 9/22, C 07 C 29/82,
C 07 C 31/02, B 01 D 3/36

(54) **Verfahren zur Trennung von Gemischen aus Paraffin bzw. Paraffinen mit 6 bis 14 C-Atomen und Alkohol bzw. Alkoholen mit 4 bis 8 C-Atomen.**

(30) Priorität: **02.02.82 DE 3203440**

(43) Veröffentlichungstag der Anmeldung:
**31.08.83 Patentblatt 83/35**

(45) Bekanntmachung des Hinweises auf die Patenterteilung:
**10.07.85 Patentblatt 85/28**

(84) Benannte Vertragsstaaten:
**BE CH DE FR GB IT LI NL SE**

(56) Entgegenhaltungen:
**EP - A - 0 010 927**
**DE - A - 1 943 379**
**FR - A - 2 118 363**
**US - A - 3 431 181**

**R. Billet, "Industrielle Destillation" (1973) Seite 229**

(73) Patentinhaber: **CHEMISCHE WERKE HÜLS AG, - RSP Patente / PB 15 - Postfach 13 20, D-4370 Marl 1 (DE)**

(72) Erfinder: **Sridhar, Srinivasan, Dr., Lehmbecker Pfad 52, D-4370 Marl (DE)**

## Beschreibung

Bei der destillativen Abtrennung flüchtiger Stoffe aus einem Stoffgemisch tritt in der Technik häufig der Fall ein, bei dem die Stoffe gleichermaßen flüchtig sind, also ein Azeotrop bilden. Dies führt dazu, daß diese Stoffe wieder im Destillat als Gemisch vorliegen. Sind zwei solcher gleichflüchtigen Stoffe in flüssigem Zustand nicht mischbar (Heteroazeotrop), so können sie leicht voneinander abgetrennt werden. Nach dem Stand der Technik ist es aber sehr schwierig, solche Stoffe im Destillat voneinander zu trennen, die miteinander mischbar sind (Homoazeotrope).

Die Trennung von homoazeotropen Gemischen aus einem $C_6$- bis $C_{14}$-Paraffin bzw. Paraffinen und einem $C_4$- bis $C_8$-Alkohol bzw. Alkoholen ist für verschiedene technische Verfahren, wie beispielsweise für die Aufarbeitung von Alkansulfonsäuren, von großem Interesse.

Die Herstellung von Alkansulfonsäuren erfolgt u. a. durch Sulfoxidation von Paraffinen in Gegenwart von Wasser und Schwefeldioxid unter Einwirkung von Licht. Nach der Reaktion trennt sich ein großer Teil der nicht umgesetzten Paraffine im Reaktoraustrag als eine Paraffinphase ab und kann unmittelbar in den Reaktor zurückgeführt werden.

Nach der Ausgasung von Schwefeldioxid- und Sauerstoffresten enthält der verbleibende Austrag Alkansulfonsäuren, Wasser, Paraffine und Schwefelsäure (Nebenprodukt).

Zur Aufarbeitung dieses Austrages sind bereits mehrere Verfahren vorgeschlagen worden. Aus der DE-OS 2 139 477 (Seite 7) (= GB-PS 1 358 095) ist es bekannt, ein sauerstoffhaltiges, polares, organisches Extraktionsmittel, das mit den Kohlenwasserstoffen nicht mischbar ist, wie Methanol, Ethanol oder niedrigmolekulare Ester, einzusetzen. Durch diesen polaren Hilfsstoff wird eine Entmischung des Gemisches in eine Paraffinphase und eine paraffinfreie, wasserhaltige, organische Phase herbeigeführt. Die letztere enthält die Sulfonsäure und Schwefelsäure. Nach Abtrennung der Restparaffine wiederum als gesonderte Phase wird sodann die Schwefelsäure durch Neutralisation mit einem alkalischen Hydroxid aus der wäßrigen Phase als Sulfat abgeschieden. Die nun vorliegenden Alkansulfonate werden destillativ als Schmelze isoliert.

Nach dem Verfahren der DE-OS 2 139 477 setzt man einen schwach polaren Alkohol mit mindestens 5 C-Atomen als Hilfsstoff ein. Hierbei werden die Sulfonsäuren und das Paraffin in die Alkoholphase überführt und die wäßrige Phase enthält die gesamte Schwefelsäure und kann als solche abgeschieden werden. Der Aufwand der Neutralisation der Schwefelsäure und der Filtration des Sulfats nach dem in der DE-OS 2 139 477 (Seite 7) beschriebenen Verfahren wird damit vermieden. Hinsichtlich der Abtrennung der Paraffine und der Wiedergewinnung des Alkohols wird zwar auch auf die Möglichkeit hingewiesen (Seite 7), daß der Alkohol und ggf. die Paraffine mit Hilfe einer dritten Komponente azeotrop abdestilliert werden können, aber die weitere Zerlegung solcher Azeotrope in einzelne Stoffe wird nicht beschrieben. Zudem führt man sogar in der späteren DE-OS 2 745 691 ( = GB-PS 1 588 363) derselben Erfinder und derselben Anmelderin gerade diesen Umstand als einen Nachteil des Verfahrens an, da der Einsatz der vorgenannten Alkohole Schwierigkeiten gäbe, weil sie eine ähnliche Flüchtigkeit aufwiesen wie die Einsatzparaffine. Bei homoazeotropen Gemischen war eine Trennung zudem, wie bereits beschrieben, nur mit Hilfe von aufwendigen Verfahren wie azeotrope Destillation unter Zusatz eines systemfremden Stoffes, Extraktion oder mehrstufige Destillation bei unterschiedlichen Drücken möglich.

So erhält man absoluten Alkohol, indem man dem wasserhaltigen Rohsprit in einer Zweisäulenapparatur ein systemfremdes Schleppmittel wie Benzol oder Trichlorethylen zusetzt (R. Billet, Industrielle Destillation [1973], Seite 229). Dieses Verfahren ist jedoch durch den Zusatz eines systemfremden Schleppmittels sehr aufwendig.

Daraus ergab sich die Aufgabe, ein Verfahren zu finden, das es ermöglicht, homoazeotrope Gemische aus einem $C_6$- bis $C_{14}$-Paraffin bzw. Paraffinen und einem $C_4$- bis $C_8$-Alkohol bzw. Alkoholen in einfacher und wirtschaftlicher Weise zu trennen.

Die Aufgabe wird erfindungsgemäß entsprechend den Angaben der Patentansprüche gelöst.

Überraschenderweise wurde gefunden, daß durch die erfindungsgemäße Führung der Stoffströme eine Trennung dieser homoazeotropen Gemische ohne Zusatz von Fremdstoffen in einzelne Ströme aus reinen Stoffen in zwei Rektifikationsschritten möglich ist. Man rektifiziert in einem Schritt in Gegenwart von Wasser als Schleppmittel. Falls ausreichende Mengen an Wasser als Schleppmittel im Zulauf nicht vorhanden sind, setzt man zusätzlich Wasser zu. Als weitere Schleppmittel sind leichtsiedende Paraffine und/oder leichtsiedende Alkohole geeignet.

Soweit höhere Paraffine mit mehr als 14 C-Atomen im Gemisch vorhanden sind, verbleiben sie fast ausschließlich im Sumpf dieser Kolonne. Das anfallende Destillat trennt man nach der Kondensation in zwei flüssige Phasen. Die dabei erhaltene organische Phase rektifiziert man in einem weiteren Schritt ohne Zusatz von Wasser und führt das Kopfprodukt, das aus einem Alkohol-Paraffin-Gemisch besteht, in den ersten Schritt zurück. Man erhält den Alkohol im unteren Teil derjenigen Kolonne, in der in der Dampfphase das Verhältnis Paraffin zu Alkohol höher ist und das Paraffin bzw. die Paraffine im unteren Teil derjenigen Kolonne, in der in der Dampfphase das Verhältnis Paraffin zu Alkohol kleiner ist.

Überraschenderweise erhält man beim erfindungsgemäßen Verfahren bei einem Azeotrop ohne Zusatz von Fremdstoffen in nur zwei Rektifikationsschritten drei Produkte in reiner Form, Paraffin bzw. Paraffine, Alkohol bzw. Alkohole und Wasser. Die erhaltenen Produkte sind infolge ihrer hohen Reinheit direkt einsetzbar. Das erhaltene Wasser kann man direkt ins Abwasser geben.

Man kann das Verfahren kontinuierlich und diskontinuierlich durchführen. Die diskontinuierliche Fahrweise eignet sich insbesondere bei kleineren Mengen. Vorzugsweise führt man das Verfahren kontinuierlich durch.

Wesentlich für die Trennung ist, daß das Mengenverhältnis von Paraffin zu Alkohol im Destillat der beiden Kolonnen unterschiedlich ist. Sollte dieses Verhältnis von Paraffin zu Alkohol in beiden Kolonnen gleich sein, so erhält man durch Veränderung des Druckes unterschiedliche Mengenverhältnisse von Paraffin zu Alkohol.

Bei dem System Ethylhexanol—$C_{10}$- bis $C_{13}$-Paraffin handelt es sich um ein homoazeotropes Gemisch. Erfindungsgemäß destilliert man das Gemisch in Gegenwart von Wasser, gegebenenfalls nach Zusatz von Wasser, beispielsweise bei 100 mbar über Kopf ab und trennt nach der Kondensation in zwei flüssige Phasen. Nach Abtrennen des Wassers aus dem Destillat als untere Phase unterwirft man die fast wasserfreie organische Phase, die aus den $C_{10}$- bis $C_{13}$-Paraffinen und dem Ethylhexanol besteht, einer weiteren Destillation und führt das Destillat in den Zulauf der ersten Destillation zurück. Bei kontinuierlicher Fahrweise erhält man im Sumpf der ersten Destillationskolonne reinen Ethylhexanol und die reinen $C_{10}$- bis $C_{13}$-Paraffine im Sumpf der zweiten Kolonne.

Ebenso handelt es sich bei dem System Hexan/Butanol um ein homoazeotropes Gemisch. Bei der erfindungsgemäßen Trennung bei 1 bar erhält man bei kontinuierlicher Fahrweise im Sumpf der einen Destillationskolonne das reine Hexan und reines Butanol im Sumpf der anderen Kolonne.

Bei $C_6$- bis $C_{14}$-Paraffingemischen kann das leichtestsiedende Paraffin bzw. können die leichtsiedenden Paraffine in der Kolonne, in der man die organische Phase destilliert, zum Teil über Kopf mit dem Alkohol übergehen. Dieses Gemisch führt man erfindungsgemäß in die vorhergehende Kolonne zurück, in der man in Gegenwart von Wasser destilliert. Im stationären Zustand fällt der gesamte Zulauf an $C_6$- bis $C_{14}$-Paraffinen im Sumpf der Kolonne an. Um diesen stationären Zustand leichter und schneller zu erreichen, setzt man vorzugsweise dem Kreislauf diese Leichtsieder zu (siehe Beispiel 3). Man kann auch leichtsiedende Paraffine zusetzen, die nicht im Einsatzparaffin enthalten sind (Beispiel 2).

Die Menge an einzusetzendem Wasser beträgt im allgemeinen 1 bis 1000, vorzugsweise 5 bis 100 Gew.-%, bezogen auf das in diesem Rektifikationsschritt vorhandene Paraffin. Gegebenenfalls führt man das nach der Destillation des Dreistoffgemisches und Kondensation des Dampfes abgetrennte Wasser ganz oder teilweise wieder in diese Destillation zurück. Ebenso kann man gegebenenfalls die nach der Trennung erhaltenen Alkohole und/oder Paraffine teilweise wieder in die Rektifikation zurückführen. Man kann auch nur die organische Phase zurückführen und die Wasserphase ausschleusen.

Man destilliert bei Normaldruck oder bei Unterdruck, bei den höheren Homologen führt man die erste Destillation vorzugsweise bei 50 bis 400 mbar, insbesondere bei 100 bis 300 mbar und die zweite Destillation vorzugsweise bei 50 bis 1013 mbar, insbesondere bei 75 bis 1013 mbar, durch.

Überraschenderweise erübrigt die erfindungsgemäße Trennung von homoazeotropen Gemischen von $C_6$- bis $C_{14}$-Paraffinen und $C_4$- bis $C_8$-Alkoholen die bisher erforderliche aufwendige Trennung wie durch Extraktion oder mehrstufige Destillation bei unterschiedlichen Drücken bzw. unter Zusatz von Fremdstoffen.

Das Verfahren ist von besonderem Interesse für die Trennung von homoazeotropen Gemischen, wie sie bei der Aufarbeitung der Alkansulfonsäuren anfallen. Das erfindungsgemäße Verfahren vereinfacht diese Aufarbeitung erheblich.

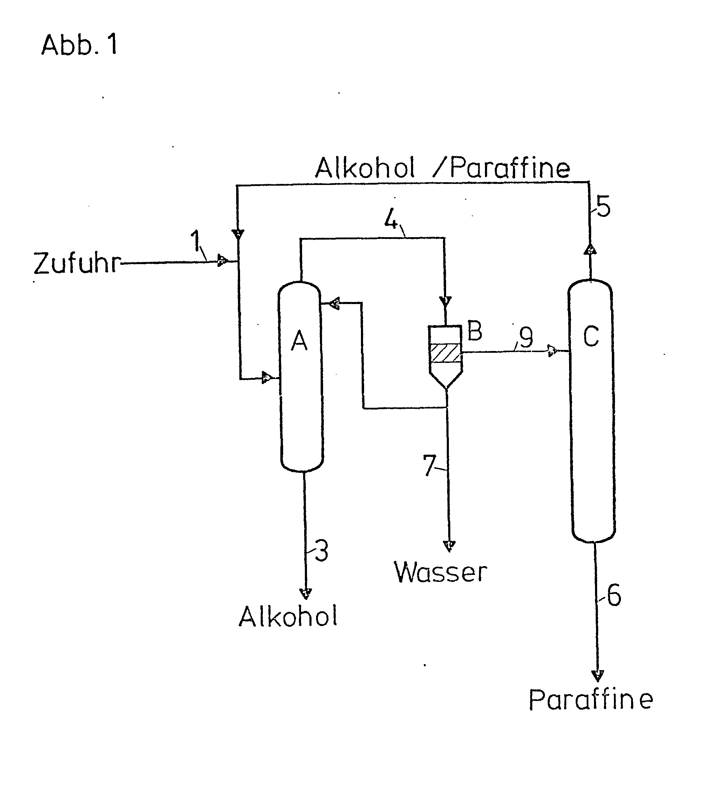

### Beispiel 1 (Abb. 1)

Auf die 6. Trennstufe (von unten) einer Kolonne A mit 8 theoretischen Trennstufen gibt man ein Gemisch aus $C_{10}$- bis $C_{13}$-Paraffinen, Ethylhexanol und Wasser der Zusammensetzung (Strom 1):

| | |
|---|---|
| n-Dekan | 43,3 kg/h |
| Undekan | 3,8 kg/h |
| Dodekan | 21,7 kg/h |
| Tridekan | 7,2 kg/h |
| Wasser | 1,0 kg/h |
| 2-Ethylhexanol | 23,0 kg/h |

Zu Beginn der Destillation füllt man den Behälter B zur Hälfte mit Wasser auf und führt es in die Kolonne A als zusätzlichen Zulauf auf die 6. Trennstufe (von unten). Beim Anfall des Destillates wird die Wasserphase aus B weiterhin vollständig zurückgeführt. Erst nachdem die Temperatur auf der 4. Trennstufe von A 80°C und auf der 7. Trennstufe 45°C erreicht hat, wird die zusätzliche Ausschleusung (Strom 7) begonnen. Ferner werden zum Beginn 383 kg/h eines Gemisches der Zusammensetzung 85 Gew.-% n-Dekan und 15 Gew.-% 2-Ethylhexanol dem Strom 1 zugemischt, und zwar nur so lange, bis in der Kolonne C die Temperatur an der 17. Stufe bzw. auf der 10. Stufe von unten auf 99°C bzw. 110°C angestiegen ist.

Man destilliert bei einem Rücklaufverhältnis von 0,5 und bei einem Druck von 100 mbar. Das Destillat, bestehend aus einem Gemisch (Strom 4) von $C_{10}$- bis $C_{13}$-Paraffinen, Wasser und Ethylhexanol hat die Zusammensetzung:

| n-Dekan | 386,6 kg/h |
|---|---|
| Undekan | 3,8 kg/h |
| Dodekan | 21,7 kg/h |
| Tridekan | 7,2 kg/h |
| Wasser | 652,7 kg/h |
| 2-Ethylhexanol | 57,9 kg/h |

(Kopftemperatur 44—45° C).

Dem Sumpf der Kolonne wird der reine Alkohol (Strom 3) entnommen.

Das Destillat der Kolonne A kondensiert man bei Normaltemperatur und gibt es in das Trenngefäß (B), worin man es in eine untere Wasserphase und eine obere organische Phase, bestehend aus $C_{10}$- bis $C_{13}$-Paraffinen und 2-Ethylhexanol, trennt. Das Wasser führt man zum Teil in die Kolonne A zurück. Der hohe Wassergehalt im Dampf der Kolonne wird auf diese Weise gewährleistet. Diejenige Wassermenge, die aber der Menge im Strom 1 entspricht, wird ausgeschleust (Strom 7).

Die organische Phase fährt man auf die 15. Stufe einer weiteren Kolonne C mit 18 Trennstufen, worin die $C_{11}$- bis $C_{13}$-Paraffine von den restlichen Stoffen bei 100 mbar/99° C am Kopf und einem Rücklaufverhältnis 3 getrennt wird. Das Kopfprodukt (Strom 5), bestehend aus 326 kg/h n-Dekan neben 58 kg/h Ethylhexanol, gibt man in den Zulaufstrom 1 der Kolonne A zurück. Dem Sumpf der Kolonne C (Strom 6) entnimmt man:

| n-Dekan | 43,3 kg/h |
|---|---|
| Undekan | 3,8 kg/h |
| Dodekan | 21,7 kg/h |
| Tridekan | 7,2 kg/h |

### Beispiel 2 (Abb. 1)

Das Gemisch (Strom 1) besteht aus:

| Undekan | 12,0 kg/h |
|---|---|
| Dodekan | 6,0 kg/h |
| 2-Ethylhexanol | 82,0 kg/h |

Die Aufarbeitung erfolgt gemäß Beispiel 1, wobei 65 kg n-Dekan/h als leichtsiedendes Hilfsmittel mit destilliert wird: Das Gemisch wird auf die 5. Stufe der Kolonne A zugeführt. Die Kolonne hat 7 Trennstufen bei einem Rücklauf von 123,22 kg/h Wasser. Die Destillation erfolgt bei 100 mbar. Der Dampf (Kopftemperatur 43—44° C) hat die Zusammensetzung:

| Dekan | 65,12 kg/h |
|---|---|
| Undekan | 12,00 kg/h |
| Dodekan | 6,00 kg/h |
| Wasser | 123,22 kg/h |
| 2-Ethylhexanol | 11,38 kg/h |

wobei der hohe Wassergehalt bzw. Dekangehalt gemäß Beispiel 1 aufrechterhalten wird, und zwar durch eine einmalige Zugabe und ohne Ausschleusung von Wasser aus dem Behälter B.

Als Kontrolltemperaturen gelten 115 bzw. 45° C auf der 2. bzw. 6. Stufe der Kolonne A und 110 bzw. 100° C auf der 5. bzw. 18. Stufe von Kolonne C. Die organische Phase fährt man auf die 13. Trennstufe der Kolonne C (insgesamt 20 Trennstufen; Rücklaufverhältnis 3; 99° C/100 mbar am Kopf). Der Dekanstrom befindet sich im Kreis zwischen dem Destillaten der Kolonne C, A und der organischen Phase im Trennbehälter B. Die Entnahme von Alkohol bzw. Paraffin erfolgt gemäß Beispiel 1.

### Beispiel 3 (Abb. 1)

Ein Gemisch der Zusammensetzung

| Undekan | 4,00 kg/h |
|---|---|
| Dodekan | 2,00 kg/h |
| 2-Ethylhexanol | 68,00 kg/h und |
| Wasser | 0,50 kg/h |

gibt man auf die 8. Trennstufe von unten einer Kolonne A mit 13 theoretischen Trennstufen (Rücklauf 24,2 kg/h Wasser) und destilliert bei 300 mbar (Kopftemperatur 68° C): Der Dampf besteht aus

| Undekan | 8,23 kg/h |
|---|---|
| Dodekan | 2,00 kg/h |
| 2-Ethylhexanol | 5,05 kg/h und |
| Wasser | 24,70 kg/h |

wobei dem System der Überschuß an Undekan einmalig, entsprechend Beispiel 1, zugesetzt wird. Es werden 4,23 kg/h Undekan und 5 kg/h 2-Ethylhexanol so lange dem Strom 1 zugeführt, bis die Temperaturen am Kopf bzw. auf der 10. Stufe von unten von Kolonne C auf 140 bis 145° C angestiegen sind. Als zusätzliche Kontrolltemperaturen gelten 140 bzw. 70° C auf der 3. bzw. 11. Stufe von Kolonne A. Den Dampf kondensiert man bei Normaltemperatur. Die Wassermenge aus dem Trennbehälter B, die der frischen Zufuhr entspricht (0,5 kg/h) wird laufend ausgeschleust. Die organische Phase wird auf die 23. Stufe (von unten) einer Kolonne mit 40 Trennstufen zugefahren und bei 300 mbar/140° C am Kopf mit einem Rücklaufverhältnis von 15 destilliert.

Die Entnahme der zugefahrenen Paraffine und dem Alkohol erfolgt gemäß Beispiel 1.

### Beispiel 4 (Abb. 1)

Ein Gemisch von Undekan und Hexanol der Zusammensetzung

| Undekan | 50,00 kg/h |
|---|---|
| n-Hexanol | 50,00 kg/h |

gibt man auf die 5. Trennstufe von unten einer Kolonne A mit 7 Trennstufen und destilliert bei 300 mbar und gemäß Beispiel 3. Zu Beginn wird dem Zulaufstrom ein Gemisch aus 14 Gew.-% Undekan und 86 Gew.-% n-Hexanol gemäß Beispiel 1 zugesetzt. Der Dampf (Kopftemperatur 67°C) hat die Zusammensetzung

| | |
|---|---|
| Undekan | 61,8 kg/h |
| Hexanol | 74,8 kg/h |
| Wasser | 190,3 kg/h |

Der Wasserschutz erfolgt wie unter Beispiel 2. (Die Rückführung der Wasserphase aus B wird beendet, wenn die Temperatur auf der 3. bzw. 6. Trennstufe von unten von Kolonne A auf 140°C bzw. 70°C angestiegen ist.) Weitere Kontrolltemperaturen sind 145 bzw. 130°C auf der 5. bzw. 30. Stufe von Kolonne C. Die Kondensation des Dampfes und Entnahme des Paraffins und Alkohols erfolgt gemäß Beispiel 1. Die Kolonne C hat 33 Trennstufen (Zulauf auf 19. Boden von unten) bei einem Rücklaufverhältnis von 10 und bei 300 mbar (Kopftemperatur 123°C). Das Destillat aus Kolonne C entspricht 11,8 kg/h Undekan, 74,8 kg/h Alkohol und ca. 1 kg/h Wasser.

### Beispiel 5 (Abb. 1)

Ein Gemisch von Undekan und Heptanol der Zusammensetzung

| | |
|---|---|
| Undekan | 85,0 kg/h |
| n-Heptanol | 15,0 kg/h |

wird ebenfalls gemäß Beispiel 4 getrennt. Zu Beginn wird dem Zulaufstrom ein Gemisch aus 38 Gew.-% Undekan und 62 Gew.-% n-Heptanol zugegeben. Die erste Destillation erfolgt bei 300 mbar, bei Bedingungen gemäß Beispiel 4. Der Dampf hat die Zusammensetzung

| | |
|---|---|
| Undekan | 146,6 kg/h |
| Heptanol | 63,6 kg/h |
| Wasser | 328,5 kg/h |

Die Kolonne C hat 44 Trennstufen bei einem Rücklaufverhältnis von 30 und bei 100 mbar (Kopftemperatur 106°C). Das Destillat entspricht 61,6 kg/h Undekan neben 63,6 kg/h Alkohole und eine geringe Menge an Wasser. Der Zulauf wird auf die 30. Trennstufe (von unten) eingefahren. Die Entnahme des Paraffins und des Alkohols erfolgt gemäß Beispiel 3. Als Kontrolltemperaturen gelten 70 bzw. 140°C auf der 2. bzw. 6. Stufe von Kolonne A und 145°C auf der 5. Stufe von Kolonne C.

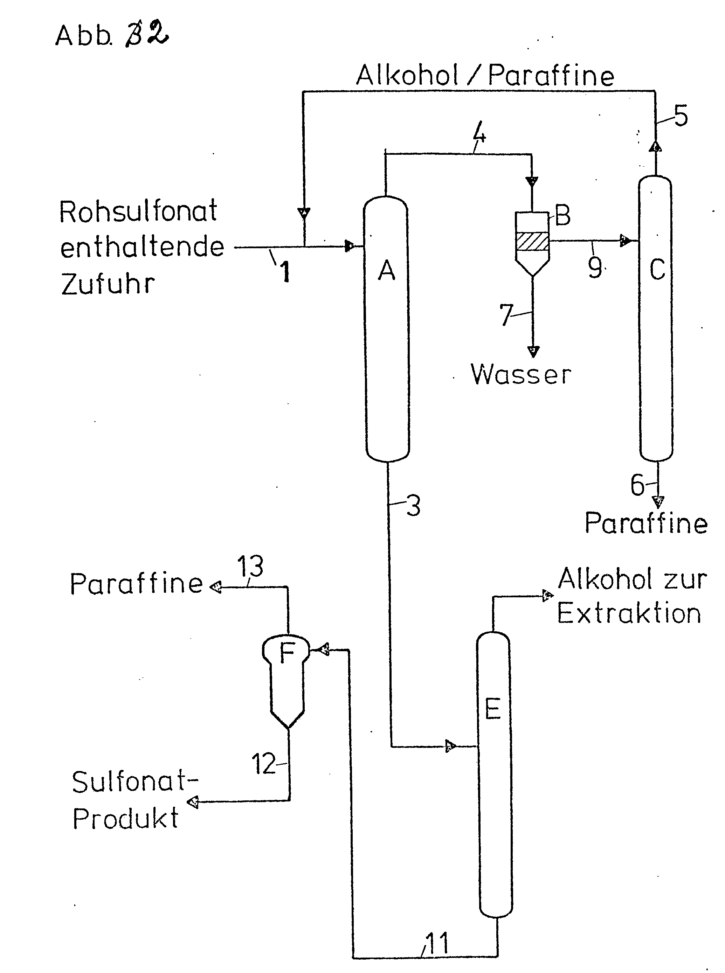

### Beispiel 6 (Abb. 2)

Der Stoffstrom enthält die Komponenten gemäß Beispiel 3 und zusätzlich

| | |
|---|---|
| Tridekan | 0,5 kg/h |
| höhere Paraffine | 0,5 kg/h |
| Alkansulfonat u. a. Salze | 16,0 kg/h |
| Wasser | 24,7 kg/h (insgesamt) |

Die Abtrennung des mitsiedenden Alkohols und leichten Paraffins erfolgt über A, B und C gemäß Beispiel 3, jedoch ohne Rückführung vom Strom 7.

Der im Sumpfstrom 3 der Kolonne A verbleibende Alkohol wird in einer weiteren Kolonne E bei 100 mbar und Rücklaufverhältnis 0,5 abdestilliert und der Extraktionsstufe zur Abtrennung von Schwefelsäure aus dem Reaktorablauf der Sulfoxidation zurückgeführt. Die Kolonne E hat 16 Trennstufen mit Zulauf auf die 12. Stufe von unten (Kopftemperatur 118°C). Die im Sumpfstrom 9 von Kolonne E verbliebenen höheren Paraffine werden in einem Dünnschichtverdampfer abdestilliert. Das Alkansulfonat-Produkt fließt aus dem Sumpf des Dünnschichtverdampfers.

### Beispiel 7 (Abb. 3)

Der Ausgangsstrom 1 entspricht dem Strom 1 vom Beispiel 6. Auf die 21. Stufe einer Kolonne A mit 26 Trennstufen gibt man das Gemisch und destilliert das Wasser über Kopf bei 300 mbar und einem Rücklaufverhältnis 1 ab. Aus der 8. Stufe unterhalb der Zulaufstelle wird der Alkohol als Seitenstrom abgelassen. Im übrigen entspricht das Beispiel dem Beispiel 1 bzw. 6.

### Beispiel 8 (Abb. 1)

Ein Gemisch der Zusammensetzung

| | |
|---|---|
| n-Hexan | 50,00 kg/h |
| t-Butanol | 50,00 kg/h |

destilliert man gemäß Beispiel 4 in Gegenwart von 40 kg/h Wasserphase im Kreislauf am Kopf der Kolonne A. Die Rückführung der Wasserphase aus B wird beendet, wenn die Temperatur auf der 7. bzw. 24. Trennstufe von unten von Kolonne A auf 83°C bzw. 70°C angestiegen ist. A hat 25 Trennstufen mit Zulauf auf die 19. Stufe von unten. Die Kolonne C hat 30 Trennstufen mit Zulauf auf die 18. Stufe von unten. Beide Destillationen erfolgen beim Normaldruck. Die Entnahme des Paraffins bzw. Alkohols erfolgt gemäß Beispiel 1. Das Paraffin ist 98%ig (2 Gew.-% Butanol). Der Alkohol ist rein (<0,3 Gew.-% Hexan). Als weitere Kontrolltemperatur gilt 65°C auf der 7. Stufe von Kolonne C.

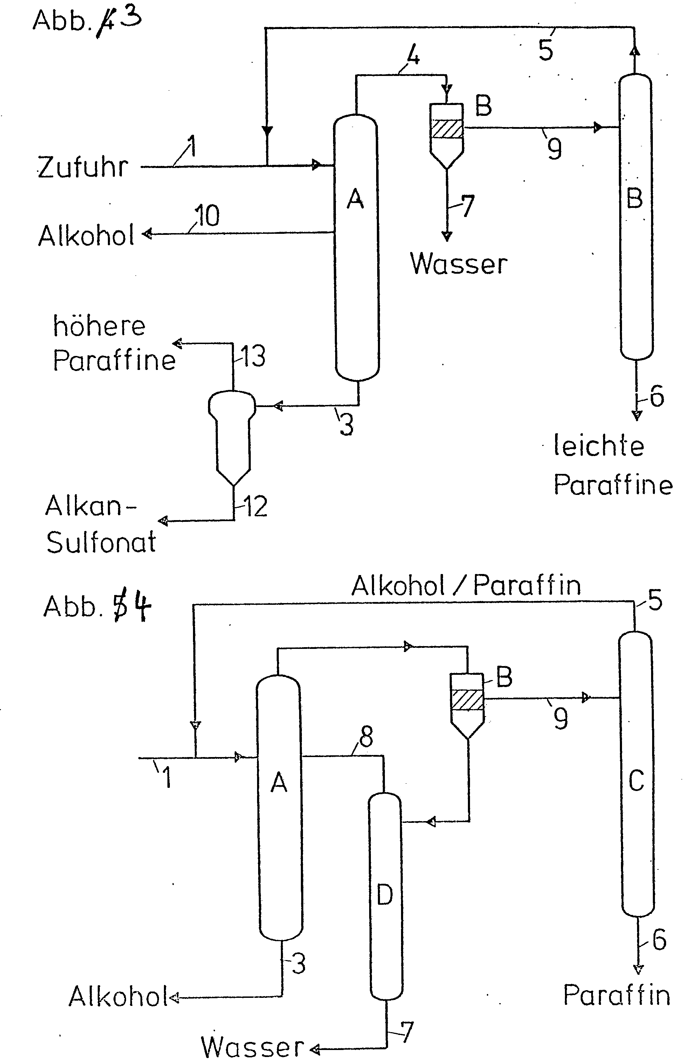

### Beispiel 9 (Abb. 4)

Ein Gemisch der Zusammensetzung

| | |
|---|---|
| n-Hexan | 30,00 kg/h |
| t-Butanol | 67,00 kg/h und |
| Wasser | 3,00 kg/h |

destilliert man gemäß Beispiel 8 (5,2 kg/h Wasser im Kreislauf am Kopf der Kolonne A). Das mit dem Zulauf zugeführte Wasser wird, von der Wasserphase in Behälter B ausgehend, über eine Strippkolonne D bekannterweise abgeschieden (Strom 7). Die Kolonne D wird beim Normaldruck (Kopftemperatur 80° C) und Rücklaufverhältnis 10 betrieben. Sie hat 25 Trennstufen mit Zulauf auf die 18. Stufe von unten. Das alkoholhaltige Destillat aus D (Strom 8) wird der Kolonne A zurückgeführt. Im übrigen entspricht das Beispiel dem Beispiel 8. Der Rücklauf der organischen Phase von der Kolonne A beträgt 604 kg/h.

### Beispiel 10 (Abb. 1)

Ein Gemisch der Zusammensetzung

| | |
|---|---|
| n-Hexan | 93,00 kg/h und |
| t-Butanol | 7,00 kg/h |

destilliert man gemäß Beispiel 8. Die Rückführung der Wasserphase aus B wird beendet, wenn die Temperatur auf der 7. Trennstufe von unten von Kolonne A auf 66° C angestiegen ist. Die Kolonne A wird beim Rücklauf der Wasserphase und zusätzlich 1120 kg/h organischer Phase (Kopftemperatur 59° C/Normaldruck) betrieben. Sie hat 35 Trennstufen mit Zulauf auf die 25. von unten. Die Verhältnisse bei Kolonne C sind 30 Trennstufen (Zulauf auf 20. Stufe), beim Rücklaufverhältnis 3 und bei den Bedingungen 32° C am Kopf/300 mbar Betriebsdruck. Im Gegensatz zu den bisherigen Beispielen und zur Abb. 2 entnimmt man das Paraffin dem Sumpf der Kolonne A und den Alkohol dem Sumpf der Kolonne C. Als zusätzliche Kontrolltemperatur gilt 56° C auf der 7. Stufe von Kolonne C.

### Beispiel 11 (Abb. 1)

Ein Gemisch der Zusammensetzung

| | |
|---|---|
| n-Oktan | 50,00 kg/h und |
| t-Butanol | 50,00 kg/h |

destilliert man in einer Vorstufe (nicht in Abbildung) — in einer Kolonne D — um nur das Homoazeotrop abzuführen. 48,6 kg/h Oktan verbleiben im Sumpf. Kolonne D hat 30 Trennstufen (Zulauf auf 17. Stufe) und hat die Betriebsbedingungen: Rücklaufverhältnis 5, Normaldruck und Kopftemperatur 82° C. Das Destillat wird gemäß Beispiel 8 in Gegenwart von 1,8 kg/h Wasserphase im Kreislauf am Kopf der Kolonne A wieder destilliert. Die Rückführung der Wasserphase aus B wird beendet, wenn die Temperatur auf der 5. Stufe von unten von Kolonne A auf 55° C angestiegen ist. Die Kolonne wird beim Rücklaufverhältnis 30 (Kopftemperatur 49° C/300 mbar) betrieben. Sie hat 50 Trennstufen mit Zulauf auf die 25. Stufe. Die Wasserphase in Behälter B ist besonders gering im Vergleich zur oberen organischen Phase. Die Verhältnisse bei Kolonne C sind 25 Trennstufen (Zulauf auf 12. Stufe) beim Rücklaufverhältnis 20 und den Bedingungen 50° C am Kopf/300 mbar Betriebsdruck. Als weitere Kontrolltemperatur gilt 70° C auf der 10. Stufe von Kolonne C.

### Beispiel 12

Einem Gemisch der Zusammensetzung

| | |
|---|---|
| n-Hexan | 50,00 kg/h und |
| $C_{5\,1}$ -Alkoholen | 50,00 kg/h |

setzt man t-Butanol laufend zu und destilliert gemäß Beispiel 8 so lange, bis die Temperaturen den Angaben von Beispiel 8 entsprechen. Danach fährt man den t-Butanol im Kreis. Die Paraffin- und $C_{5\,1}$ -Alkohol-Entnahme erfolgt gemäß Beispiel 8.

### Patentansprüche

1. Verfahren zur Trennung von homoazeotropen Gemischen aus einem Paraffin bzw. Paraffinen mit 6 bis 14 C-Atomen und einem Alkohol bzw. Alkoholen mit 4 bis 8 C-Atomen in Gegenwart eines Schleppmittels in zwei Rektifikationsschritten, dadurch gekennzeichnet, daß man in einem ersten Schritt in Gegenwart von Wasser als Schleppmittel rektifiziert, das anfallende Destillat nach Kondensation in zwei flüssige Phasen trennt, die dabei erhaltene organische Phase in einem weiteren Schritt ohne Zusatz von Wasser rektifiziert und das Kopfprodukt, das aus einem Alkohol/Paraffin-Gemisch besteht, in den ersten Schritt zurückführt, wobei man das Paraffin bzw. die Paraffine und den Alkohol bzw. die Alkohole im unteren Teil der jeweiligen Rektifikationsschritte erhält und das gegebenenfalls im Ausgangsgemisch vorhandene Wasser ausschleust.

2. Verfahren nach Anspruch 1, dadurch gekennzeichnet, daß man zusätzlich Wasser als Schleppmittel einsetzt.

3. Verfahren nach Anspruch 1, dadurch gekennzeichnet, daß man zusätzlich leichtsiedende Paraffine als Schleppmittel einsetzt.

4. Verfahren nach Anspruch 1, dadurch gekennzeichnet, daß man leichtsiedende Alkohole als Schleppmittel einsetzt.

5. Verfahren nach Ansprüchen 1 bis 4, dadurch gekennzeichnet, daß man die nach der Trennung erhaltenen Stoffe Wasser, Alkohol und/oder Paraffin ganz oder teilweise wieder in die Rektifikation zurückführt.

### Claims

1. A process for the separation of homoazeotropic mixtures of one or more paraffins of 6 to 14 carbon atoms and one or more alcohols of 4 to 8 carbon atoms in two rectification stages in the presence of an entrainer, characterised in that rectification in the first stage is carried out in the presence of water as the entrainer, the resulting distillate is separated into two liquid phases after condensation, the organic phase thus obtained is rectified in a further stage without the addition of water and the overhead product consisting of an alcohol/paraffin mixture is recycled to the first stage, so that the paraffin(s) and the alcohol(s) are obtained in the lower portions of the respective rectification columns and the water which is optinally present in the starting mixture is removed.

2. A process according to claim 1, characterised in that additional water is introduced as entrainer.

3. A process according to claim 1, characterised in that additional low-boiling paraffin is introduced as entrainer.

4. A process according to claim 1, characterised in that low-boiling alcohol is introduced as entrainer.

5. A process according to any of claims 1 to 4, characterised in that the water, alcohol and/or paraffin obtained by the separation are wholly or partly recycled to the rectification.

## Revendications

1. Procédé de séparation de mélanges homoazéotropes comprenant une paraffine ou des paraffines contenant de 6 à 14 atomes de carbone et un alcool ou des alcools contenant de 4 à 8 atomes de carbone, en présence d'un agent d'entraînement, en deux étapes de rectification, caractérisé par le fait que, dans une première étape, on rectifie en présence d'eau comme agent d'entraînement, que l'on sépare le distillat obtenu, après condensation, en deux phases liquides, que l'on rectifie dans une autre étape, sans addition d'eau, la phase organique ainsi obtenue et que l'on recycle à la première étape le produit de tête qui est formé d'un mélange d'alcool et de paraffine, en obtenant ainsi la paraffine ou les paraffines et l'alcool ou les alcools dans la partie inférieure des étapes de rectification considérées, et qu'on élimine l'eau qui est éventuellement présente dans le mélange initial.

2. Procédé selon la revendication 1, caractérisé par le fait que l'on utilise complémentairement de l'eau comme agent d'entraînement.

3. Procédé selon la revendication 1, caractérisé par le fait que l'on utilise complémentairement, comme agent d'entraînement, des paraffines très volatiles.

4. Procédé selon la revendication 1, caractérisé par le fait que l'on utilise, comme agent d'entraînement, des alcools très volatils.

5. Procédé selon les revendications 1 à 4, caractérisé par le fait que l'on renvoie à nouveau totalement ou partiellement à la rectification les corps obtenus après la séparation, à savoir l'eau, l'alcool et/ou la paraffine.

Abb. 1

Abb. 32

Alkohol / Paraffine

Rohsulfonat
enthaltende
Zufuhr

**1**

A

4

B

**5**

C

9

7

Wasser

**3**

6

Paraffine

Paraffine

13

F

Alkohol zur
Extraktion

12

E

Sulfonat-
Produkt

11

**0 086 890**

Abb. 13

Zufuhr

Alkohol

höhere
Paraffine

Alkan-
Sulfonat

Wasser

leichte
Paraffine

Abb. 14

Alkohol / Paraffin

Alkohol

Wasser

Paraffin

13